# EUROPÄISCHE PATENTSCHRIFT

(11) **EP 2 068 755 B1**
(45) Veröffentlichungstag und Bekanntmachung des Hinweises auf die Patenterteilung: **11.01.2012**
(21) Anmeldenummer: 07817491.9
(22) Anmeldetag: 11.09.2007
(51) Int. Cl.: A61F 2/00

(54) **Einstückiges Stomaunterstützungsimplantat und ein Verfahren zu dessen Herstellung**
Integrally formed stoma-supporting implant and production method
Implant de support du ventre en une seule pièce et son procédé de production

(30) Priorität: 12.09.2006 DE 102006043500; 11.10.2006 DE 102006048476
(43) Veröffentlichungstag der Anmeldung: 17.06.2009
(73) Patentinhaber: FEG Textiltechnik Forschungs- und Entwicklungsgesellschaft MbH, 52070 Aachen (DE)
(72) Erfinder: BERGER, Dieter, 76532 Baden-Baden (DE); OBOLENSKI, Boris, 52070 Aachen (DE); KLINGE, Uwe, 4850 Montzen (BE); SCHNEEMELCHER, Stefan, 52064 Aachen (DE)
(74) Vertreter: Gottschald, Jan
(86) Internationale Anmeldenummer: PCT/DE2007/001618
(87) Internationale Veröffentlichungsnummer: WO 2008/031411

(56) Entgegenhaltungen:
- WO-A-2004/010897
- WO-A-2007/034145
- US-A- 6 017 355
- US-A1- 2005 021 058

## Beschreibung

Die Erfindung betrifft ein einstückiges Stomaunterstützungsimplantat und ein Verfahren zu dessen Herstellung .

Die operative Reparation eines Gewebebruchs mit der Folge einer Hernie wird heutzutage überwiegend mit einer plastischen Prothese durchgeführt. Hierbei werden netzartige oder folienartige Flächenstrukturen eingesetzt, die entweder aus einer Konstruktion von textilen Mono- oder Multifilamenten oder aber aus durch geeignete Extrusionsverfahren hergestellte Folien unterschiedlicher Dicke hergestellt werden.

Die flächigen Strukturen werden zur Unterstützung der Gewebestruktur an denjenigen Stellen im Körper eingesetzt, an denen der Eingeweidebruch entweder durch eine natürliche oder aber durch eine operativ bedingte Pforte auftritt. Während die Entstehung eines Eingeweidebruchs an den natürlichen Pforten der Bauchdecke von einer Vielzahl von unterschiedlichen biologischen Faktoren abhängt, resultieren die operativ bedingten Brüche in der Regel aus einer ungünstig verheilten Operationsnarbe.

Die grundlegende Aufgabe einer plastischen Prothese in Form einer flächigen Netz- oder Folienstruktur besteht bei allen bekannten Operationsverfahren zur Behebung eines Eingeweidebruchs in der Verstärkung der umliegenden natürlichen Gewebestrukturen. Während an einer natürlichen Pforte wie beispielsweise einem Samenstrang der natürliche Bauchdeckendurchgang jedoch nicht verschlossen werden darf, so dass die Prothese in diesem Fall immer eine Öffnung aufweisen muss, ist bei einer postoperativen Bruchsituation wie bei einer Narbenhernie in der Regel ein Verschließen des Gewebedurchgangs notwendig, die Prothese darf also keine Öffnung haben.

In allen bekannten Fällen ist es notwendig, einerseits den Gewebedefekt durch eine Verstärkung mit Hilfe einer Prothese zu beheben. Andererseits darf diese Prothese nicht zu neuen Komplikationen fuhren. Diese Prothese soll eine ausreichend hohe Festigkeit in allen Belastungsrichtungen aufweisen, um einen erneuten Bruch zu verhindern. Um die natürliche Elastizität des umgebenden Gewebes nach der Implantation nicht zu beschränken, ist eine dem Gewebe ähnliche Elastizität in allen Bewegungsrichtungen notwendig. Nur wenn diese beiden Grundvoraussetzungen erfüllt sind, kann die Prothese mittel- und langfristig ohne Komplikationen ihre Aufgabe erfüllen.

Eine besondere Form des Gewebebruchs stellt die Parastomalhernie dar. Durch die Schaffung eines künstlichen Durchgangs durch die Bauchdecke für einen neuen Darmausgang wird die Bauchdecke an einer hierfür nicht geeigneten Stelle durchbrochen. Der neu geschaffene Durchgang durch die Bauchdecke entspricht in kleinster Weise den Bedingungen eines natürlichen Durchgangs. Es werden somit bei der Schaffung eines Stomas die hinsichtlich der Entstehung einer Hernie ungünstigen Bedingungen geschaffen. Wissenschaftliche Untersuchungen zeigen, dass sich statistisch in über 90 % aller Stomaoperationen in der Folgezeit an dieser Stelle eine Hernie ergibt.

In vielen Fällen wird eine Parastomalhernie durch eine erneute Operation wiederum mit einer flächigen netzförmigen oder folienartigen Prothese in einer ähnlichen Art und Weise operiert, wie dies beispielsweise bei einem Leistenbruch der Fall ist. Hierzu wird ähnlich wie bei einem Leistenbruch in die flächige Netz-oder Folienstruktur eine Öffnung mittig in die Fläche geschnitten. Um die Netz-oder Folienstruktur um den künstlichen Darmausgang herumzulegen, wird ein weiter Schnitt von der Kante bis zur aufgeschnittenen Mittelöffnung vorgenommen. Durch das Überlappen der Bereiche rechts und links dieses Schnitts wölbt sich die Mitte der Netz- oder Folienstruktur auf, und das Implantat wird dreidimensional.

Die US 2005/0043716 A1 zeigt eine implantierbare Prothese, bei welcher eine Kanüle in das Gewebe eingebracht wird. Um die Kanüle herum wird eine zweidimensionale Prothese an der Innenseite des Gewebes angeordnet. Sodann wird der Darm durch den von der Kanüle geschaffenen Gang nach außen gezogen. Sodann wird die Kanüle entfernt. Im Patienten verbleiben das flächige Implantat sowie Nähte zum Fixieren des Darms am Gewebe und am Implantat. Optional können segmentierte Klappen am Implantat gelenkig gelagert angeordnet sein. Die US 4,584,316 zeigt ein dreiteiliges Implantat mit einem flächigen Innenstück einem hieran angenähten Zylinderstück und einem hieran angenähten zweiten flächigen Stück, wobei ein seitlicher Eingang zum Einbau des Implantats vorgesehen ist.

Die DE 100 19 604 A1 zeigt ein Implantat, welches eine ebene Basis mit einem Gestrick oder Gewirk hat, wobei in einem zentralen Bereich der ebenen Basis ein Maschenmuster inhomogen, nämlich deutlich flexibler, gestaltet ist, damit die flächige Basis im zentralen Bereich zu einem Vorsprung ins Dreidimensionale verformt werden kann. In dem sich ergebenden Pilzkopf soll eine Verstärkung vorgesehen werden.

Die DE 698 21 936 T2 zeigt ein Implantat, welches beispielsweise für den Darm verwendet werden soll. Es besteht aus einem hohlen Zylinder und einer daran mehrstückig angesetzten ebenen Kontaktfläche. Ein Flantsch gibt dem Implantat Stabilität.

Kasperk, Willis, Klinge und Schumpelick beschreiben in Chirurg 2002-73:895-898 verschiedene Verfahren zum Operieren von parastomalen Hernien.

WO 2004/010897 A1 zeigt ein Implantat, bei dem eine Mehrzahl von langgestreckten Netzelementen an einem mittigen Ringelement befestigt sind. Da die Netzelemente als separates Teil an dem mittigen Ringelement befestigt sind, ist dieses Implantat weder einstückig noch verbindungsfrei. Bei einer weiteren Ausführungsform eines in dieser Druckschrift gezeigten Implantats sind die langgestreckten Netzelemente einstückig an einem mittigen inneren Ringelement angebracht. Dieses mittige Ringelement weist eine zentrische Durchgangsöffnung auf, wobei die Dicke dieses Ringelements maximal 1 mm oder weniger beträgt.

US 2005/0021058 A1 zeigt ein Implantat mit den Merkmalen des Oberbegriffs von Anspruch 1 bzw. Anspruch 5. Bei einem einstückigen verbindungsfreien

Implantat nach dieser Druckschrift ist aus einer ebenen Basis heraus ein schlauchartiger Ansatz geformt, der an seinem freien Ende geschlossen ist.

Der Erfindung liegt die Aufgabe zu Grunde, die bestehende Technologie zu verbessern.

Zur Lösung der vorstehend genannten Aufgabe ist ein erfindungsgemäßes Stomaunterstützungsimplantat einstückig und verbindungsfrei ausgebildet, und weist eine ebene Basis und einen schlauchartigen Ansatz auf, wobei die Basis und der schlauchartige Ansatz eine Netzstruktur aufweisen. Der schlauchartige Ansatz ist offen und bildet somit einen Durchlass, wobei die Kreuzungspunkte der Fäden, welche die Maschen der Netzstruktur definieren, so fixiert sind, dass die Maschen in der Größe unverändert bleiben. Weiterhin sind in dem Implantat Poren vorgesehen, die eine Mindestgröße von 0,6 mm aufweisen.

Die Erfindung wird den Ansprüchen definiert.

Begrifflich sei hierzu zunächst erläutert, dass sich das vorgeschlagene Implantat von denjenigen aus der DE 698 21 936 T2 schon dadurch unterscheidet, dass das hier vorgeschlagene Implantat einstückig ist, was es deutlich kostengünstiger in der Herstellung und ebenfalls im Handling macht. Gegenüber den herkömmlichen Implantierverfahren mit aufgeschnittenen Netzen besteht der große Vorteil, dass das hier vorgeschlagene Implantat verbindungfrei ist, insbesondere also nahtfrei, klebefrei und verschweißnahtfrei. Immer dann, wenn zwei Flächenstücke miteinander in beliebiger Weise verbunden werden, also beispielsweise durch Verkleben, Verschweißen, Vernähen oder andere Fügeverfahren, entstehen an der Stelle, an die zu fügenden Teile zusammenkommen, gegenüber der übrigen Basisfläche unterschiedliche Materialeigenschaften. Wenn die Gesamtstruktur des Implantats anschließend belastet wird, kommt es im Bereich der Fügung in Vergleich zum übrigen Bereich zu einer ungleichmäßigen Lastverteilung, die dazu führen kann, dass entweder eine notwendige physiologische Elastizität nicht erreicht wird oder dass eine Überlastung eintritt, die zu einer Zerstörung der Strukturen führt. Nur wenn an Übergangsstellen eine kontinuierliche und nahtfreie Materialverbindung besteht, können diese Probleme vermieden werden.

"Als die "ebene Basis" ist der im Wesentlichen zweidimensionale Teil des Implantats zu verstehen. Dies kann beispielsweise eine Gewirk, ein Gestrick, ein Netz, ein Gewebe oder eine andere Struktur aufweisen. Der "offene schlauchartige Durchlass" besteht aus einem schlauchartigen Ansatz, der sich aus der Basis heraus erhebt und - meist nach einem Übergangsbereich - in einen Schlauch übergeht. Dieser soll am von der Basis entfernten Ende offen sein. Hierdurch dient er als Durchlass für den Darm oder ein anderes Gefäß.

Die Eigenschaft der Einstückigkeit und der Verbindungsfreiheit soll sich nicht nur auf die Basis als solche beschränken, sondern gleichfalls auf den Übergang von der Basis zum Ansatz sowie vom Ansatz zum schlauchartigen Bereich. Auf diese Weise wird ein kontinuierlicher Übergang von der Öffnung zum Darm erreicht. Anders als bei bisherigen Implantaten, die einlagig und verbindungsfrei vorlagen, nämlich nur aus einem eingeschnittenen Netz bestanden, ist nun in der als Basis dienenden Netzplastik die Öffnung so ausgestaltet, dass auch ein Teilstück des am Stoma anliegenden Darms mit von der Netzplastik berücksichtigt wird.

Es versteht sich, dass ein dergestalt geformtes Implantat nicht seitlich um den Darm eingelegt werden kann, sondern dass der Darm durch dieses Implantat hindurchgeführt werden muss. Ein grundlegender Gedanke der vorliegenden Erfindung ist darin zu sehen, dass die Prothese bereits bei derjenigen Operation, bei der das Stoma angelegt wird, direkt mit implantiert wird. Die überaus hohe Komplikationsrate von über 90 % deutet der Erfinder als einen Hinweis darauf, dass in fast allen Fällen mit dem Auftreten einer Parastomalhernie gerechnet werden muss. Ein grundlegender Gedanke des Vorschlags, ein Implantat wie vorgeschlagen auszugestalten, liegt darin, dass eine geeignete Plastik bei der Erstoperation prophylaktisch eingesetzt werden soll.

Die Aufgabe wird weiterhin durch ein erfindungsgemäßes Verfahren zum Herstellen eines einstückigen verbindungsfreien Stomaunterstützungsimplantats gelöst. Bei diesem Verfahren wird aus einer ebenen Basis mittels eines Umformverfahrens ein schlauchartiger Ansatz geformt, wobei die Basis und der schlauchartige Ansatz eine Netzstruktur aufweisen. Der schlauchartige Ansatz wird zu einem Durchlass geöffnet, wobei Kreuzungspunkte der Fäden, welche die Maschen der Netzstruktur definieren, so fixiert sind, dass die Maschen in der Größe unverändert bleiben, wobei in dem Implantat Poren gebildet werden, die eine Mindestgröße von 0,6 mm aufweisen.

So lässt sich aus einer ebenen Basis bei geeigneter Gestaltung sehr kostengünstig durch ein Umformverfahren der offene schlauchartige Durchlass formen, indem zunächst ein Ansatz geformt wird und dieser geöffnet wird, so dass sich ein offener schlauchartiger Durchlass ergibt.

Demgemäß löst ein weiterer Aspekt, der jedoch nicht zur vorliegende Erfindung gehört, die Aufgabe mit einem Verfahren zum Herstellen eines Stomaunterstützungsimplantats, wobei aus einer ebenen Basis mittels eines Umförmverfahrens ein schlauchartiger Ansatz geformt und dieser geöffnet wird.

Durch Umformverfahren bleibt das Implantat frei von jeglichen nachträglichen Verbindungen wie insbesondere Nähten oder Klebeverbindungen, was die mechanische Kompatibilität im Gewebe erheblich heraufsetzt.

In vorteilhafter Weiterbildung der vorliegenden Erfindung ist die ebene Basis mit homogenen Flächeneigenschaften, insbesondere mit einem homogenen Maschenmuster versehen.

Begrifflich sei zunächst erläutert, dass eine "homogene Flächeneigenschaft" bereits dann vorliegen soll, wenn die Flächeneigenschaft hinsichtlich einer Richtung innerhalb der Basis homogen ist. Hierbei sei insbesondere an eine Hauptwirkrichtung der Basis gedacht, so insbesondere an eine der beiden Richtungen der Fadenverläufe bei einem Gewebe oder bei anderen Fadenkonstruktionen, bei denen sich mindestens eine Hauptstreckungsrichtung der Fäden feststellen lässt.

Vor allem sei jedoch daran gedacht, dass die Flächeneigenschaften der Basis hinsichtlich zweier, vorzugsweise orthogonal zueinander liegender Richtungen jeweils homogen sind oder dass sogar isotrope Flächeneigenschaften vorliegen.

Hinsichtlich der Flächeneigenschaft sei insbesondere an die Dehnfestigkeit und/oder eine Maschen- und/oder Porenweite gedacht. So liegt ein homogenes Maschenmuster dann vor, wenn entlang einer Hauptmaschenrichtung die Maschen eine gleiche Größe haben. Von besonderem Vorteil ist es, wenn alle Maschen gleich groß sind.

Wenn aus einer Basis mit homogenen Flächeneigenschaften ein schlauchartiger Ansatz geformt wird, der sogleich oder später zu einem offenen schlauchartigen Durchlass geöffnet werden kann, so entsteht ein Implantat mit auch hinsichtlich der dritten Dimension relativ gleichmäßigen mechanischen Eigenschaften. Zwar wird es in der Regel in einem Übergangsbereich zwischen der Basis und dem schlauchförmigen Stück zu einer Inhomogenisierung der Flächeneigenschaften, insbesondere des Maschenmusters, bezüglich der ursprünglichen Basis kommen. Versuche des Erfinders haben jedoch ergeben, dass insbesondere der schlauchförmige Bereich wieder zu einem sehr ähnlichen mechanischen Verhalten geformt werden kann.

Die Erfindung unterscheidet sich in diesem Bezug wesentlich von den Vorschlägen aus der DE 100 19 604 A1. Dort wird ausdrücklich gefordert, dass ein inhomogenes Maschenmuster vorliegen soll, konkret ein stark geschwächtes Maschenfeld mit größeren Maschen im zentralen Bereich, aus welchem der geschlossene Ansatz geformt werden sollt. Die vorliegende Erfindung geht diesbezüglich einen anderen Weg und hat sich bei ersten Prototypen als sehr erfolgreich erwiesen.

In vorteilhafter Weiterbildung des erfindungsgemäßen Verfahrens wird der schlauchartige Ansatz mittels eines Umformverfahrens aus einer Basis mit homogenen Flächeneigenschaften geformt, insbesondere aus einer Basis mit einem homogenen Maschenmuster.

Die Vorteile eines solchen Verfahrens ergeben sich unmittelbar aus den vorstehenden Erläuterungen.

In vorteilhafter Weiterbildung der Erfindung kann auch der schlauchartige Ansatz mit homogenen Flächeneigenschaften, insbesondere mit einem homogenen Maschenmuster, versehen sein.

Nach diesem Aspekt der Erfindung weisen entweder die Basis und/oder der schlauchartige Ansatz im dreidimensionalen Zustand ein homogene Maschenmuster auf.

Im Gegensatz dazu wird es sich bei dem Implantat nach der DE 100 19 604 A1 in der Praxis nicht vermeiden lassen, dass der geschwächte Bereich mit großen Maschen zum Teil als Basisfläche verbleibt, erst dann in einen Übergangsbereich übergeht, und dass auch am schlauchförmigen Bereich erweiterte Maschen und somit eine geschwächte Struktur vorliegt. Dies vermeidet der sechste Aspekt der Erfindung.

Im Idealfall ist die Flächeneigenschaft, insbesondere das Maschenmuster, sowohl an der Basis als auch am schlauchartigen Bereich und ebenfalls am Übergangsbereich homogen. In der Praxis wird es sich gerade bei einem Herstellungsverfahren mit einer Umformung aus einem ursprünglichen zweidimensionalen Flächenstück nur mit großen Anstrengungen vermeiden lassen, dass die Maschen am Übergangsbereich eine konstante Größe behalten. Es ist jedoch von Vorteil, wenn das Maschenmuster zumindest außerhalb eines Übergangsbereichs zwischen einer Ebene und einem Zylinder homogen ist.

Es wurde bereits erläutert, dass der Ansatz zu einem Durchlass geöffnet ist.

Die Basis und der schlauchartige Ansatz weisen eine Netzstruktur auf. Eine Netzstruktur kennzeichnet sich dadurch, dass aus Fäden Maschen gebildet sind, wobei Kreuzungspunkte der Fäden, welche die Maschen definieren, so fixiert sind, dass die Maschen in der Größe unverändert bleiben. Ansonsten bestände die Gefahr, dass sich Fäden verschieben, dabei die Maschen einengen und somit das lebende Gewebe einschnüren.

Das Stomaunterstützungsimplantat ist bzw. bevorzugt thermofixiert. Insbesondere eignet sich für die Herstellung des vorgeschlagenen Implantats in Form eines parastomalen Netzes eine Kombination aus einem mechanischen und einem thermischen Umformverfahren. Hierzu kann eine flächige Netz- oder Folienstruktur in einen geeigneten Rahmen eingespannt und das zu formende Ansatzstück durch einen Tiefziehstempel in die Netzebene eingedrückt werden. Im verformten Zustand wird anschließend eine thermische Behandlung durchgeführt durch welche die Verformung im Netz fixiert wird.

Neben den mechanischen Anforderungen, die sowohl nach einer ausreichenden Festigkeit als auch nach einer möglichst physiologisch verträglichen Elastizität verlangen, stellt die Biokompatibilität einer alloplastischen Prothese einen entscheidenen Faktor für den langfristigen Erfolg einer Operation dar. Die Biokompatibilität, die als möglichst komplikationsfreie Zellreaktion auf das Eindringen eines Implantatwerkstoffs in den menschlichen Körper definiert ist, wird mikroskopisch durch die Eigenschaft der Implantatoberfläche sowie makroskopisch durch den strukturellen Aufbau der Flächenstruktur bestimmt.

Im mikroskopischen Maßstab wird die Biokompatibilität maßgebend durch die primäre Zellreaktion der körpereigenen Proteine auf die Molekularstruktur der Implantatoberfläche bestimmt. In Ahängigkeit der initialen Reaktionen der Körperzellen baut sich auf der Oberfläche des Implantats eine Schicht aus körpereigenen Zellen auf. Die Dicke und die molekulare Zusammensetzung dieser Schicht sind maßgebende Faktoren für die Integration des Implantats in das umgebende Körpergewebe. Nur wenn diese Schicht eine ausreichend gute Verbindung zwischen dem Implantat und dem umgebenden körpereigenem Gewebe herstellt, kann die grundlegende Funktion einer Gewebeverstärkung durch das Implantat sichergestellt werden.

Im Lichte dessen wird vorgeschlagen, dass das Stomaunterstützungsimplantat PVDF oder modifiziertes PVDF aufweist. Aus neueren wissenschaftlichen Ergebnissen ist bekannt, dass besonders fluorhaltige Polymere wie beispielsweise PVDF (Polyvinylidenfluorid) eine sehr positive Biokompatibilität besitzen. Ein anderer Implantatwerkstoff, der eine wissenschaftlich belegte gute Biokompatibilität besitzt, ist beispielsweise Polypropylen.

Es wurde bereits angesprochen, dass als Umformverfahren bevorzugt Tiefziehen eingesetzt ist bzw. wird.

Ein hiermit hergestelltes Implantat hat bei geeigneter Gestaltung nur eine, in sich geschlossene Durchgangsöffnung: Ein zusätzlicher Schnitt vom Seitenrand zur Mittelöffnung ist nicht notwendig. Hierdurch wird eine zusätzliche Schwächung der Implantatstruktur verhindert. Gleichzeitig entfällt das zusätzliche Schließen dieser Schnittkante mit einer Naht. Das Implantat kann während der Stomaoperation prophylaktisch implantiert werden.

Insbesondere mittels Tiefziehen kann mit einfachen Mitteln eine aus der Basisebene herausragende zylinderförmig Struktur geschaffen werden.

Es wird vorgeschlagen, dass ein schlauchartiger Ansatz eine Länge von zwischen etwa 5 mm und etwa 50 mm aufweist.

Unabhängig hiervon wird vorgeschlagen, dass ein schlauchartiger Ansatz einen Durchmesser von zwischen etwa 10 mm und etwa 40 mm aufweist.

Diese Werte haben sich bei Versuchen des Erfinders als sehr vorteilhaft herausgestellt.

Es wurde bereits erläutert, dass die Biokompatibilität eines Implantats zunächst aus der zellulären Reaktion körpereigener Zellen auf die molekulare Zusammensetzung der Implantatoberfläche resultiert.

Des Weiteren muss berücksichtigt werden, dass durch die Ausbildung einer Zellschicht auf der Implantatoberfläche keine geschlossen Zellschicht entstehen darf. Dieses Verhalten ist immer dann zu beobachten, wenn die Implantatstruktur einerseits offenporig gestaltet ist, wenn andererseits jedoch die Abstände in den Poren nicht ausreichend groß sind.

Die Mindestgröße einer Pore, bei der ein Zusammenwachsen der Zellschicht als Reaktion auf die Implantatoberfläche nicht erfolg, hängt von der Dicke der Zellschicht ab. Diese Dicke wiederum ergibt sich aus dem Material bzw. aus der molekularen Zusammensetzung der Oberfläche des Implantats. In jedem Falle muss die Größe der Pore in alle betrachteten Richtungen ausreichend groß sein.

Ist die Mindestgröße nur in einer Dimension erreicht, so kann bei einem zu niedrigen Abstand der beispielsweise hierzu senkrecht liegenden Dimension dennoch ein komplettes Zusammenwachsen von gegenüberliegenden Zellschichten in Folge der Implantatreaktionen erfolgen. Der Mindestabstand ist somit in allen Dimensionen einzuhalten, und bevorzugt auch in allen Belastungssituationen des Implantats.

Besonders bei einer eindimensionalen Dehnung kommt es bei netzartigen Strukturen zu einer Verzerrung der ursprünglichen geometrischen Form der Poren. Durch die Elongation in einer Richtung entsteht in der Regel eine Kontraktion in der zur Dehnungsrichtung rechtwinklig liegenden Richtung. Hierdurch wird der ursprüngliche Abstand der Porenränder verringert. Da es aber die Hauptaufgabe eines solchen Implantats darstellt, durch Krafteinfluss injizierte Dehnungen aufzunehmen, um das umgebende Gewebe zu stützen, muss die Forderung nach einer minimalen Porengröße besonders unter Belastungsbedingungen eingehalten werden.

Insbesondere wird vorgeschlagen, dass mehr als 40 % aller Poren der Implantatfläche eine Porengröße aufweisen, die in allen Dimensionen einen Mindestabstand von gegenüberliegenden Porenrändern von mindestens 0,6 mm aufweisen.

Mit einer solchen Mindestgröße insbesondere unter physiologischen Belastungen ist eine Porengröße gegeben, die genügend groß ist, um ein Aneinanderwachsen der Zellschichten in Folge der Gewebereaktion auf das Implantat zu vermeiden.

Auch unabhängig von sämtlichen vorstehend genannten Eigenschaften ist bevorzugt ein schlauchartiger Ansatz vorhanden, der vier Scharen von bogenförmigen, umeinander liegenden Maschen-/ oder Porenreihen aufweist. Eine solche Geometrie hat sich bei Versuchen mit Tiefziehstempeln an Prototypen ergeben.

Nach einem siebten Aspekt der Erfindung löst die gestellte Aufgabe die Verwendung eines Stomaunterstützungsimplantats, zum Unterstützen des Stomas beim Anlegen des Stomas. In diesem Falle wird das Implantat prophylaktisch implantiert, was die geschriebenen Vorteile für den Patienten hat.

Die Erfindung wird nachstehend anhand zwei Ausführungsbeispiele unter Bezugnahme auf die Zeichnung näher erläutert. Funktional gleiche Bauelemente können gleiche Bezugsziffern tragen. Es zeigen:
- Fig 1: schematisch in einer räumlichen Ansicht ein Stomaunterstützungsimplantat mit einer Basis und mit einem zentralen Durchgang.
- Fig. 2: schematisch in einer räumlichen Ansicht eine beispielhafte Ausführung eines netzförmigen, offenporigen Implantats und
- Fig.3: fotografisch einen offen Durchlass an einem als Prototyp hergestellten Implantat.

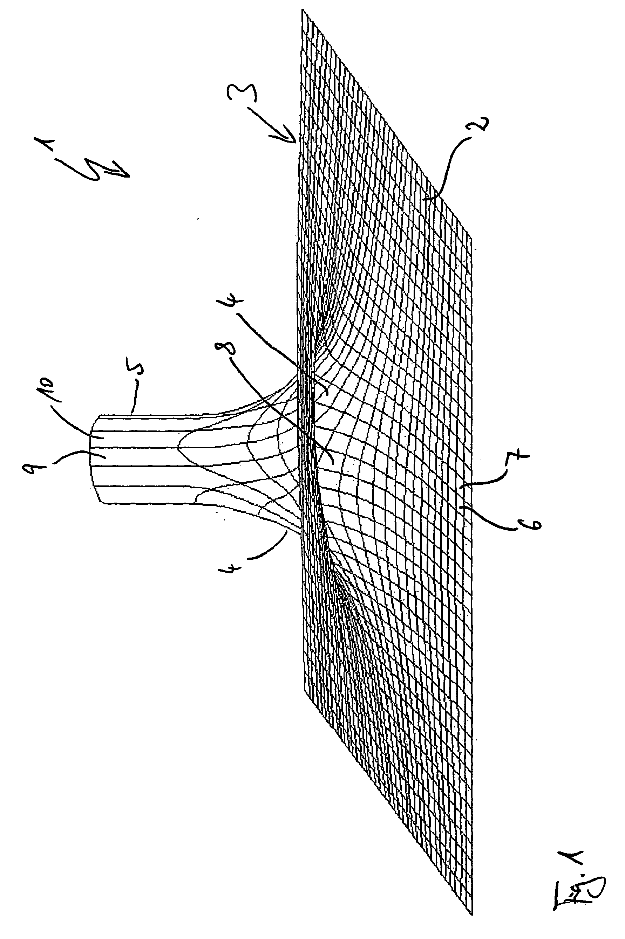

Das Implantat 1 in Figur 1 besteht aus einem einstückigen, nahtfreien netzartigen Gewirk aus Fäden 2 (exemplarisch gekennzeichnet) aus PVDF.

Eine flächige Basis 3 geht an einem Übergangsbereich 4 aus einer zweidimensionalen Struktur in einen offenen schlauchartigen Durchlass 5 über. Der Übergangsbereich 4 zwischen der Basis 3 und dem schlauchartigen Durchlass 5 ist nahtfrei und kontinuierlich. Im Herstellverfahren wurden sowohl der Übergangsbereich 4 als auch der schlauchartige Durchlass aus der flächigen Basis 3 heraus erzeugt. Das gesamte Implantat 1 war ursprünglich ein zweidimensionales Netz.

Der schlauchartige Durchlass 5 dient als zylindrischer Stutzen zum Durchführen des Darms oder eines anderen Gefäßes. Der Durchlass ist nicht - wie bei herkömmlichen Netzplastiken - an die flächige Basis 3 zusätzlich angefügt. Stattdessen ist der Übergangsbereich 4 als runder Übergang das Ergebnis einer kontinuierlichen Umformung der flächigen Netzstruktur der Basis 3 aus der Ebene heraus.

Durch diese Konstruktion wird einerseits ein vollkommen nahtfreier Übergang zwischen der Flächenebene der Basis 3 und dem zylindrischen Ansatzstutzen des Durchlasses 5 realisiert. Andererseits wird die Elastizität sowohl im flächigen Bereich der Basis 3 als auch im zylindrischen Bereich des Durchlasses 5 möglichst wenig beeinträchtigt. Poren 6, 7 (exemplarisch gekennzeichnet) der Basis 3 sind bis an den Rand des Übergangsbereichs 4 heran im gesamten flächigen Bereich der Basis 3 gleich groß. Erst im Übergangsbereich 4 kommt es zu verzerrten Poren 8 (exemplarisch gekennzeichnet). Am schlauchartigen Durchlass 5 sind zwar anders geformte Poren 9, 10 (exemplarisch gekennzeichnet) als an der Basis 3. Diese sind aufeinander bezogen jedoch zumindest hinsichtlich eines Umfangs des schlauchartigen Durchlasses 5 homogen.

Dennoch haben sowohl die Basis 3 als auch der Durchlass 5 und der Übergangsbereich 4 im Wesentlichen dieselbe Elastizität.

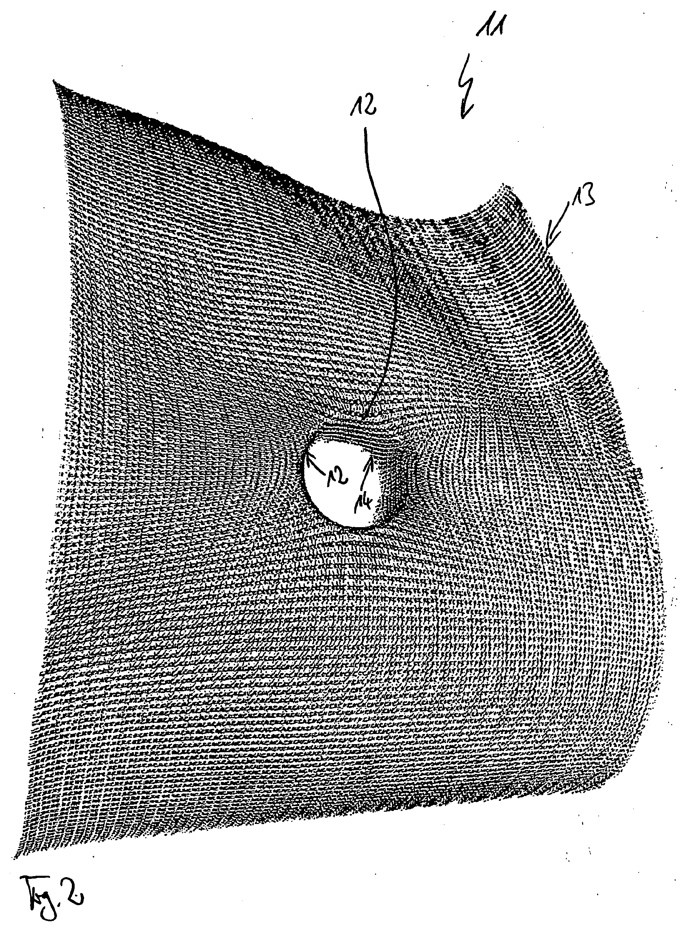

Das alternativ ausgeführte Implantat 11 in Figur 2 ist mit dem Implantat 1 aus Figur 1 sehr vergleichbar.

Ein Übergangsbereich 12 ist jedoch mit einem erheblich kleineren Radius ausgebildet, so dass beim Implantat 11 zwar von einer Basis 13 zu einem schlauchartigen Durchlass 14 ein sehr schneller Übergang erfolgt. Der Übergang ist aber dennoch rund ausgestaltet und absolut kanten- und nahtfrei. Gleiches gilt sowohl für die Basis 13 als auch für den schlauchartigen Durchlass 14.

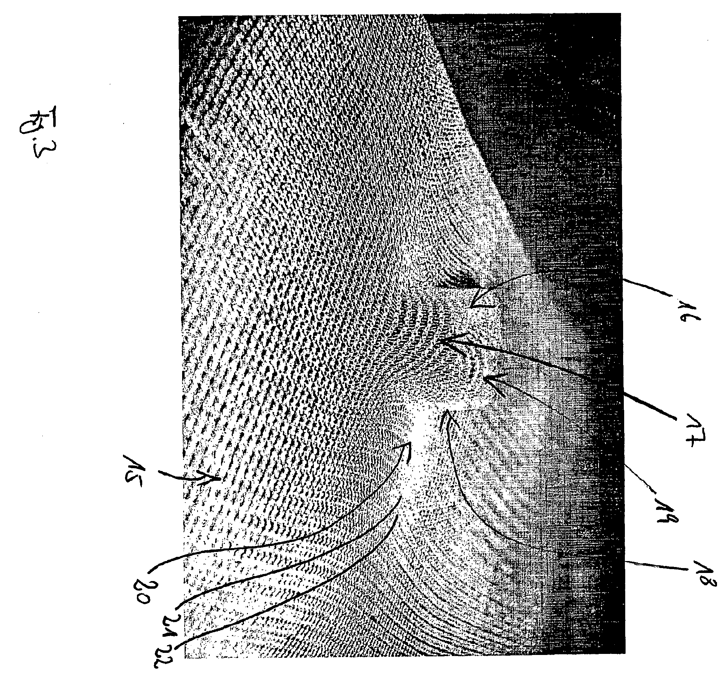

Beim fotografisch dargestellten Implantat 15 in Figur 3 zeigen sich dieselben geometrischen Verhältnisse wie beim Implantat 11 aus Figur 2.

Am schlauchartigen Durchlass 16 sind vier Scharen 17, 18, 19 (eine vierte Schar ist verdeckt und kaum sichtbar) von bogenförmig umeinander liegenden Porenreihen ausgebildet. Die Poren sind jeweils untereinander etwa gleich groß.

Auch in einem Zulaufbereich 20 an der Basis 15 zeichnet sich ein bogenförmige Muster von Porenreihen 21, 22 (exemplarisch gekennzeichnet) ab. Dennoch sind die Poren auch hier mit einer nur geringen Verzerrung gleich groß.

## Patentansprüche

1. Einstückiges, verbindungsfreies Stomaunterstützungsimplantat (1) mit einer ebenen Basis (3) und mit einem schlauchartigen Ansatz (5), wobei die Basis (3) und der schlauchartige Ansatz (5) eine Netzstruktur aufweisen, **dadurch gekennzeichnet, dass** der schlauchartige Ansatz (5) offen ist und somit einen Durchlass bildet, wobei Kreuzungspunkte der Fäden, welche die Maschen der Netzstruktur definieren, so fixiert sind, dass die Maschen in der Größe unverändert bleiben, und dass Poren (6, 7) mit einer Mindestgröße von 0,6 mm vorgesehen sind.

2. Stomaunterstützungsimplantat (1) nach Anspruch 1, bei welchem der offene, schlauchartige Durchlass (5) durch ein Umformverfahren aus der ebenen Basis geformt ist.

3. Stomaunterstützungsimplantat (1) nach Anspruch 1 oder 2, bei dem die ebene Basis (3) und/oder der schlauchartige Ansatz (5) mit homogenen Flächeneigenschaften, insbesondere mit einem homogenen Maschenmuster versehen sind.

4. Stomaunterstützungsimplantat nach einem der vorhergehenden Ansprüche, **dadurch gekennzeichnet, dass** das Maschenmuster außerhalb eines Übergangsbereichs zwischen einer Ebene und einem Zylinder homogen ist.

5. Verfahren zum Herstellen eines einstückigen, verbindungsfreien Stomaunterstützungsimplantats (1), wobei aus einer ebenen Basis mittels eines Umformverfahrens ein schlauchartiger Ansatz (5) geformt wird, wobei die Basis (3) und der schlauchartige Ansatz (5) eine Netzstruktur aufweisen, **dadurch gekennzeichnet, dass** der schlauchartige Ansatz zu einem Durchlass (5) geöffnet wird, wobei Kreuzungspunkte der Fäden, welche die Maschen der Netzstruktur definieren, so fixiert sind, dass die Maschen in der Größe unverändert bleiben, wobei Poren (6, 7) in dem Implantat gebildet werden, die eine Mindestgröße von 0,6 mm aufweisen.

6. Verfahren nach Anspruch 5, wobei die Basis (3) homogene Flächeneigenschaften und insbesondere ein homogenes Maschenmuster aufweist.

7. Stomaunterstützungsimplantat bzw. Herstellverfahren nach einem der vorhergehenden Ansprüche, **dadurch gekennzeichnet, dass** das Stomaunterstützungsimplantat (1) thermofixiert ist bzw. wird.

8. Stomaunterstützungsimplantat bzw. Herstellverfahren nach einem der vorhergehenden Ansprüche, **dadurch gekennzeichnet, dass** das Stomaunterstützungsimplantat (1) PVDF oder modifizierte PVDF aufweist.

9. Stomaunterstützungsimplantat bzw. Herstellverfahren nach einem der vorhergehenden Ansprüche, **dadurch gekennzeichnet, dass** als Umformverfahren Tiefziehen eingesetzt ist bzw. wird.

10. Stomaunterstützungsimplantat bzw. Herstellverfahren nach einem der vorhergehenden Ansprüche, **dadurch gekennzeichnet, dass** ein schlauchartiger Ansatz eine Länge von zwischen 5 mm und etwa 50 mm aufweist.

11. Stomaunterstützungsimplantat bzw. Herstellverfahren nach einem der vorhergehenden Ansprüche, **dadurch gekennzeichnet, dass** ein schlauchartiger Ansatz einen Durchmesser von zwischen etwa 10 mm und etwa 40 mm aufweist

12. Stomaunterstützungsimplantat nach einem der vorhergehenden Ansprüche, **dadurch gekennzeichnet, dass** ein schlauchartiger Ansatz vier Scharen (17, 18, 19) von bogenförmigen, umeinander liegenden Maschen- oder Porenreihen aufweist.

## Claims

1. Integrally formed, connectionless stoma-supporting implant (1) with a plane base (3) and with a tubular protrusion (5), wherein the base (3) and the tubular protrusion (5) have a net structure, **characterized, in that** the tubular protrusion (5) is open and thus forms a passage, wherein intersection points of the threads that define the meshes of the net structure are fixed such that the meshes remain unchanged in size, and **in that** pores (6, 7) with a minimum size of 0.6 mm are provided.

2. Stoma-supporting implant (1) according to Claim 1, in which the open, tubular passage (5) is formed from the plane base by a shaping process.

3. Stoma-supporting implant (1) according to Claim 1 or 2, in which the plane base (3) and/or the tubular protrusion (5) are/is provided with homogeneous surface properties, in particular with a homogeneous mesh pattern.

4. Stoma-supporting implant according to one of the preceding claims, **characterized in that** the mesh pattern is homogeneous outside a transition area between a plane and a cylinder.

5. Method for producing an integrally formed, connectionless stoma-supporting implant (1), wherein a tubular protrusion (5) is formed from a plane base by means of a shaping process, wherein the base (3) and the tubular protrusion (5) have a net structure, **characterized in that** the tubular protrusion is opened to form a passage (5), wherein intersection points of the threads that define the meshes of the net structure are fixed such that the meshes remain unchanged in size, wherein pores (6, 7) are formed in the implant that have a minimum size of 0.6 mm.

6. Method according to Claim 5, wherein the base (3) has homogeneous surface properties and in particular a homogeneous mesh pattern.

7. Stoma-supporting implant and production method according to one of the preceding claims, **characterized in that** the stoma-supporting implant (1) is thermofixed.

8. Stoma-supporting implant and production method according to one of the preceding claims, **characterized in that** the stoma-supporting implant (1) has PVDF or modified PVDF.

9. Stoma-supporting implant and production method according to one of the preceding claims, **characterized in that** thermoforming is used as the shaping process.

10. Stoma-supporting implant and production method according to one of the preceding claims, **characterized in that** a tubular protrusion has a length of between 5 mm and approximately 50 mm.

11. Stoma-supporting implant and production method according to one of the preceding claims, **characterized in that** a tubular protrusion has a diameter of between approximately 10 mm and approximately 40 mm.

12. Stoma-supporting implant according to one of the preceding claims, **characterized in that** a tubular protrusion has four sets (17, 18, 19) of arc-shaped rows of meshes or pores lying around one another.

## Revendications

1. Implant (1) de soutien de stoma, d'un seul tenant et exempt de liaison, présentant une base plane (3) dotée d'un appendice (5) en forme de tuyau flexible, la base (3) et l'appendice (5) en forme de tuyau flexible présentant la structure d'un treillis,
**caractérisé en ce que**
l'appendice (5) en forme de tuyau flexible est ouvert et forme ainsi un passage, les points de contact des fils qui définissent les mailles de la structure en treillis étant immobilisés de telle sorte que la taille des mailles reste inchangée et
**en ce que** des pores (6, 7) d'une taille minimale de 0,6 mm sont prévus.

2. Implant (1) de soutien de stoma selon la revendication 1, dans lequel le passage (5) ouvert en forme de tuyau flexible est formé par déformation de la base plane.

3. Implant (1) de soutien de stoma selon lés revendications 1 ou 2, dans lequel la base plane (3) et/ou l'appendice (5) en forme de tuyau flexible sont dotés de propriétés de surface homogènes et en particulier d'un motif de mailles homogène.

4. Implant de soutien de stoma selon l'une des revendications précédentes, **caractérisé en ce que** le motif de mailles est homogène à l'extérieur d'une partie de transition entre un plan et un cylindre.

5. Procédé de fabrication d'un implant (1) de soutien de stoma, d'un seul tenant et exempt de liaison, dans lequel un appendice (5) en forme de tuyau flexible est formé à partir d'une base plane au moyen d'un procédé de façonnage, la base (3) et l'appendice (5) en forme de tuyau flexible présentant une structure en treillis, **caractérisé en ce que**
l'appendice en forme de tuyau flexible est ouvert pour former un passage (5), les points de croisement des fils qui définissent les mailles de la structure en treillis étant immobilisés de telle sorte que la taille des mailles reste inchangée, des pores (6, 7) dont la taille minimale est de 0,6 mm étant formés dans l'implant.

6. Procédé selon la revendications 5, dans lequel la base (3) présente des propriétés de surface homogènes et en particulier un motif de mailles homogène.

7. Implant de soutien de stoma ou procédé de fabrication selon l'une des revendications précédentes, **caractérisé en ce que** l'implant (1) de soutien de stoma est thermofixé.

8. Implant de soutien de stoma ou procédé de fabrication selon l'une des revendications précédentes, **caractérise en ce que** l'implant (1) de soutien de stoma présente du PVDF ou du PVDF modifié.

9. Implant de soutien de stoma ou procédé de fabrication selon l'une des revendications précédentes, **caractérisé en ce que** le procédé de déformation utilisé est un emboutissage.

10. Implant de soutien de stoma ou procédé de fabrication selon l'une des revendications précédentes, **caractérisé en ce qu'**un appendice en forme de tuyau flexible présente une longueur comprise entre 5 mm et environ 50 mm.

11. Implant de soutien de stoma ou procédé de fabrication selon l'une des revendications précédentes, **caractérisé en ce qu'**un appendice en forme de tuyau flexible présente un diamètre compris entre environ 10 mm et environ 40 mm.

12. Implant de soutien de stoma selon l'une des revendications précédentes, **caractérisé en ce qu'**un appendice en forme de tuyau flexible présente quatre ensembles (17, 18, 19) de rangées de mailles ou de pores disposées en arc de cercle les unes autour des autres.
